# FASCICULE DE BREVET EUROPEEN

(11) **EP 2 874 604 B1**
(45) Date de publication et mention de la délivrance du brevet: **14.02.2018**
(21) Numéro de dépôt: 13747452.4
(22) Date de dépôt: 17.07.2013
(51) Int. Cl.: A61K 9/08, A61K 9/107, A61K 47/18

(54) **UTILISATION D'UN AGENT CHELATANT AMPHOTERE POUR PREVENIR LES ALLERGIES DE CONTACT**
VERWENDUNG EINES AMPHOTEREN CHELATBILDNERS ZUR VERMEIDUNG VON KONTAKTALLERGIEN
USE OF AN AMPHOTERIC CHELATING AGENT FOR PREVENTING CONTACT ALLERGIES

(30) Priorité: 18.07.2012 FR 1256939
(43) Date de publication de la demande: 27.05.2015
(73) Titulaire: PREVOR INTERNATIONAL, 75015 Paris (FR)
(72) Inventeur: BLOMET, Joël, F-95760 Valmondois (FR); MATHIEU, Laurence, F-33400 Talence (FR); MEYER, Marie-Claude, F-75015 Paris (FR)
(74) Mandataire: Cabinet Plasseraud
(86) Numéro de dépôt international: PCT/FR2013/051718
(87) Numéro de publication internationale: WO 2014/013195

(56) Documents cités:
- WO-A2-01/93858
- US-A- 5 763 486

## Description

### Domaine de l'invention

La présente invention se rapporte au domaine des produits de prévention des allergies de contact.

### Contexte de l'invention

WO01/93858 A2 décrit l'utilisation d'un complexe d'aluminium et de ligand éthylènediaminetétraacétique pour la prévention et/ou le traitement des urticaires provoquées par les agents urticants choisis dans le groupe comprenant les méduses, les orties, le mancellinier, le sumac vénéneux et les piqûres d'insectes.

Les allergies de contact, et notamment l'allergie due au contact avec des objets ou dispositifs en caoutchouc ont fortement augmenté ces dernières années. Bien qu'habituellement modérés, les symptômes peuvent dans certains cas avoir de lourdes conséquences. Les allergies peuvent en effet être de deux types :
- immédiate avec urticaire, rhinite, conjonctivite, asthme et risque d'anaphylaxie (état de choc et /ou asphyxie ;
- retardée avec dermite de contact allergique (eczéma).

Les allergies de contact se développent par la répétition du contact avec des substances allergènes.

L'allergie au caoutchouc, et en particulier au latex, plus particulièrement au latex naturel, touche 1 à 5 % de la population. Elle touche plus particulièrement les personnes exposées à des contacts répétés au caoutchouc, comme les personnes travaillant dans l'univers médical. Ainsi, 5 % à 22 % du personnel médical, infirmières, médecins, chirurgiens, kinésithérapeutes, podologues, développent des allergies au caoutchouc. Les malades qui ont subi de nombreuses opérations sont également susceptibles de développer des allergies au latex, tout comme les personnels des laboratoires, des salons de coiffure qui utilisent des gants en caoutchouc, les personnes qui utilisent les méthodes prophylactiques des préservatifs et les personnels de l'industrie du caoutchouc.

L'allergie au caoutchouc représente la 2e cause de choc anaphylactique au cours d'une anesthésie. L'allergie au caoutchouc naturel (LN) a été reconnue comme un problème de santé publique depuis la fin des années 1980.

Le caoutchouc naturel ou latex est une émulsion aqueuse de gouttelettes sphériques de polyisoprène enveloppées d'une couche de protéines hydrosolubles. Il peut être d'origine synthétique ou naturel. Sous forme naturelle, il provient de la sève de l'*Hevea brasiliensis.* Il est très présent dans les objets de la vie quotidienne. A titre d'exemples d'objets en latex ou comprenant du latex qui sont donc de nature à provoquer des réactions allergiques on peut citer les gants de ménage, les pneus, les ballons, les tétines, les tubes de plongée, les palmes, les lunettes de natation, les bonnets de bains, les colles, les manches grips de raquette de tennis, les préservatifs, le matériel médical : bandages élastiques, seringues avec joints en caoutchouc, sondes, alèses, tubulures de perfusion, gouttières pour soins dentaires et gants d'examens ou de chirurgiens, etc.

La meilleure façon de prévenir les allergies de contact est d'éviter l'utilisation et/ou le contact avec des objets contenant les substances allergènes. Ainsi, pour prévenir les allergies au caoutchouc, il est recommandé de ne pas utiliser d'objets en caoutchouc.

Il existe des produits de substitution, notamment en polyuréthane, polyvinyle, nitrile, etc. permettant la substitution des objets ou dispositifs en caoutchouc, mais ceux-ci s'avèrent beaucoup plus onéreux et, pour ce qui est des doigtiers, gants, préservatifs, ils affectent la sensibilité au toucher. Il existe donc un réel besoin en un produit permettant de prévenir les allergies de contact et notamment les allergies au caoutchouc.

De façon surprenante et inattendue les présents inventeurs ont trouvé qu'un agent chélatant amphotère particulier permettait de prévenir les allergies de contact.

### Résumé de l'invention

D'une façon générale, la présente invention porte sur l'utilisation d'au moins un agent chélatant amphotère qui comprend un complexe à base d'aluminium et d'acide éthylène diamine tétraacétique ou son sel trisodique présentant la formule générale [Al(Y)Bn]^{c'}D_{c} avec B représentant OH⁻, BO₂⁻, H⁺, Y représentant un tétracarboxylate pouvant être protoné quatre fois pour former l'acide éthylène diamine tétraacétique, n représentant un nombre entier égal à 0, 1, 2 ou 3, D étant un contre-ion, de préférence Na⁺, c étant un nombre entier égal à 0, 1, 2 ou 3 et c' étant un nombre relatif ayant la même valeur absolue que c, pour prévenir les allergies de contact qui sont provoquées par le contact répété avec la peau et/ou les muqueuses de substances allergènes, ledit agent chélatant amphotère étant stabilisé par un acide aminé choisi dans le groupe consistant en la glycine, l'histidine, l'arginine, la phénylalanine, l'isoleucine, la leucine, la méthionine, la proline, la valine, le tryptophane, la sérine, la glutamine, la cystine et leurs mélanges. Selon un autre aspect, la présente invention porte sur un dispositif comportant une partie en matériau allergène, notamment en latex, destinée à entrer en contact avec la peau ou les muqueuses, qui est recouverte par une couche contenant au moins un agent chélatant amphotère tel que mentionné ci-dessus.

Selon encore un autre objet, la présente invention porte sur l'utilisation d'une composition comprenant au moins ledit agent chélatant amphotère mentionné précédemment et au moins un excipient, pour prévenir les allergies de contact.

### Description Détaillée de l'Invention

La présente invention porte sur l'utilisation d'au moins un agent chélatant amphotère qui comprend un complexe à base d'aluminium et d'acide éthylène diamine tétraacétique ou son sel trisodique présentant la formule générale [Al(Y)Bn]^{c'}D_{c} avec B représentant OH⁻, BO₂⁻, H⁺, Y représentant un tétracarboxylate pouvant être protoné quatre fois pour former l'acide éthylène diamine tétraacétique, n représentant un nombre entier égal à 0, 1, 2 ou 3, D étant un contre-ion, de préférence Na⁺, c étant un nombre entier égal à 0, 1, 2 ou 3 et c' étant un nombre relatif ayant la même valeur absolue que c, pour prévenir les allergies de contact, en particulier, les allergies au caoutchouc, ledit agent chélatant amphotère étant stabilisé par un acide aminé choisi dans le groupe consistant en la glycine, l'histidine, l'arginine, la phénylalanine, l'isoleucine, la leucine, la méthionine, la proline, la valine, le tryptophane, la sérine, la glutamine, la cystine et leurs mélanges. Dans la présente invention, par « allergies de contact » on entend toutes les allergies qui sont provoquées par le contact répété avec la peau et/ou les muqueuses de substances allergènes. A titre d'allergènes présents dans des objets ou dispositifs susceptibles d'entrer en contact avec la peau et/ou les muqueuses et qui provoquent des allergies de contact, on peut citer, les protéines du latex, des métaux lourds tels que Al, As, Cd, Cr, Hg, Ni, Pb, Sr et Te, le disulfure de tétraméthylthiuram, le 2-mercaptobenzothiazole, le carbamate de benzyle, le bisphénol, l'acrylate d'éthyle, l'araldite 506 epoxy Resin, le méthanal, les allergènes de type protéinique (tels que les profilines, les tropomyosines, les LTP (les protéines de transfert de lipides), les bet v 1-like protéines PR-10, les polcalcines, les bêta parvalbumines, les 2s albumines, les bêta expansines, les polygalacturonases, les Ag5 (antigènes 5), les albumines, les caséines, les lipocalines, le groupe 5 des graminées, les 11s globulines, les 7s vicilin-like globulines, le groupe 4 des graminées, les papaïne-like protéases à cystéine, les phospholipases A1, les inhibiteurs de protéase à sérine, les hyaluronidases, les chitinases de classe 1, les thaumatin-like protéines, etc.) et leurs mélanges.

L'invention est particulièrement appropriée pour la prévention des allergies au caoutchouc, et notamment au caoutchouc naturel. Dans la présente invention, par « allergies au caoutchouc » on entend les allergies provoquées par les protéines présentes dans le caoutchouc naturel dérivé de l'arbre *Hevea brasiliensis,* mais aussi par d'autres substances allergènes présentes dans le caoutchouc. A titre d'exemples de telles protéines allergènes, on peut citer l'hévéine, la prohévéine, la profiline, le facteur d'allongement du caoutchouc, l'endo-1,3-β-glucosidase, etc. D'autres exemples de substances allergènes non protéiniques présentes dans le caoutchouc sont les agents de vulcanisation (thiurames, dithiocarbamates, benzothiazoles, guanidines, et thiourées), agents antioxydants ou antiozonants (dérivés de la PPD - phénylène paradiamine- et quinolines), phénols (hydroquinones), phosphites... ou autres additifs tels que digluconate de chlorhexidine, sels de chlorure de diméthyl didécyl ammonium, isothiazolinones, formaldéhyde, métaux divers, etc...

De telles allergies de contact se manifestent par une dermite de contact, une urticaire de contact, un eczéma, des rhinites, des conjonctivites, de l'asthme, un choc anaphylactique.

Dans le texte de la présente demande, par « allergies de contact » ou « allergies au caoutchouc » on désignera indifféremment l'allergie et ses manifestations.

L'agent chélatant amphotère de formule générale [Al(Y)Bₙ]^{c'}D_{c} utilisé selon l'invention peut être formé de préférence par une combinaison quasi-stoechiométrique de l'ion aluminium Al³⁺, du ligand Y et d'un agent stabilisant choisi parmi OH⁻, BO₂⁻ ou H⁺. En conséquence, son pH reste neutre, le plus petit de ses pK acides est dans la gamme de 6 à 10 alors que le plus grand de ses pK basiques est dans la gamme de 5 à 8, et le plus grand pK basique est inférieur au plus petit pK acide.

Selon un mode de réalisation particulier, ledit complexe à base d'aluminium et d'Y est stabilisé par une base faible telle que les acides aminés choisis dans le groupe comprenant la glycine, l'histidine, l'arginine, la lysine, la phénylalanine, l'alanine, l'isoleucine, la leucine, la méthionine, la proline, la valine, le tryptophane, la sérine, la glutamine, la cystine et leurs mélanges. La glycine est tout à fait appropriée.

Selon l'invention, l'utilisation dudit agent chélatant amphotère est réalisée de telle sorte qu'il soit en contact avec la peau et/ou les muqueuses.

L'efficacité d'utilisation de l'agent chélatant amphotère conforme à l'invention a été testée par des analyses morphologiques basées sur la théorie suivante : Les cellules de Langerhans sont des cellules dendritiques que l'on retrouve dans l'épiderme et qui contiennent des granules de Birbeck. Ils existent normalement dans des ganglions lymphatiques et dans la peau au niveau du stratum spinosum de l'épiderme. Ces cellules spécialisées dans la captation des antigènes s'activent lorsque la peau est mise en contact avec un allergène. Cela présente un premier signe du début d'un processus de sensibilisation. Des méthodes d'immuno-marquage, notamment des récepteurs de surface CD1a des cellules de Langerhans permettent d'en mesurer le nombre et d'observer leur migration de l'épiderme au derme. La composition préventive de l'invention est efficace si l'antigène est stoppé, c'est-à-dire non capté par les cellules de Langerhans épidermiques. Celles-ci ne vont donc pas migrer vers le derme. L'efficacité de la composition selon l'invention se mesure donc par le nombre de cellules de Langerhans non migrantes par centimètre d'épiderme. Il n'a pas été observé de migration sensible du nombre de ces cellules après mise en contact de la zone de peau couverte par la composition selon l'invention avec des agents allergènes et notamment avec les protéines allergènes du caoutchouc naturel telles que l'hévéine.

L'invention porte également sur un dispositif comportant une partie en matériau susceptible de contenir des allergènes destinée à entrer en contact avec la peau ou les muqueuses, qui est recouverte par une couche contenant au moins un agent chélatant amphotère qui comprend un complexe à base d'aluminium et d'acide éthylène diamine tétraacétique ou son sel trisodique présentant la formule générale [Al(Y)Bₙ]^{c'}D_{c} avec B représentant OH⁻, BO₂⁻, H⁺, Y représentant un tétracarboxylate pouvant être protoné quatre fois pour former l'acide éthylène diamine tétraacétique, n représentant un nombre entier égal à 0, 1, 2 ou 3, D étant un contre-ion, de préférence Na⁺, c étant un nombre entier égal à 0, 1, 2 ou 3 et c' étant un nombre relatif ayant la même valeur absolue que c.

L'invention est particulièrement adaptée aux dispositifs en caoutchouc ou comportant une partie en caoutchouc destinée à entrer en contact avec la peau et/ou les muqueuses.

Des exemples de dispositif selon l'invention sont les préservatifs, les gants, les doigtiers, les pansements et compresses, les dispositifs médicaux tels que sondes, tubulures, dispositifs de drainage, de perfusion, dispositifs médicaux utiles en urologie, dispositifs médicaux de l'abord respiratoire, etc.

Selon un mode de réalisation particulier, au moins la partie du dispositif destiné à entrer en contact avec la peau et/ou les muqueuses, est recouverte d'un film comprenant ledit agent chélatant amphotère.

Ledit film est de préférence une préparation lubrifiante.

Dans le cas des préservatifs, à la fois la surface externe et la surface interne peuvent être recouvertes d'une préparation lubrifiante comportant ledit agent chélatant amphotère de façon à prévenir les deux partenaires des allergies au latex.

L'invention porte également sur une composition comprenant au moins un agent chélatant amphotère qui comprend un complexe à base d'aluminium et d'acide éthylène diamine tétraacétique ou son sel trisodique présentant la formule générale [Al(Y)Bₙ]^{c'}D_{c} avec B représentant OH⁻, BO₂⁻, H⁺, Y représentant un tétracarboxylate pouvant être protoné quatre fois pour former l'acide éthylène diamine tétraacétique, n représentant un nombre entier égal à 0, 1, 2 ou 3, D étant un contre-ion, de préférence Na⁺, c étant un nombre entier égal à 0, 1, 2 ou 3 et c' étant un nombre relatif ayant la même valeur absolue que c, et au moins un excipient, pour une utilisation pour prévenir les allergies de contact et en particulier les allergies au caoutchouc, caractérisée en ce que ledit agent chélatant amphotère est stabilisé par un acide aminé choisi dans le groupe consistant en la glycine, l'histidine, l'arginine, la phénylalanine, l'isoleucine, la leucine, la méthionine, la proline, la valine, le tryptophane, la sérine, la glutamine, la cystine et leurs mélanges. La composition peut se présenter sous forme d'émulsion huile dans eau ou eau dans huile, sous forme de gel, en particulier de gel hydrophile ou gel de silicone.

Ledit excipient présent dans la composition est un excipient de qualité pharmaceutique ou cosmétique.

Des exemples d'excipients pouvant être présents dans la composition selon l'invention sont choisis parmi un agent gélifiant, des tensio-actifs, des cires ou huiles synthétiques ou naturelles, des agents hydratants et émollients, des agents anti-perspirants, des additifs améliorant la texture tels que des conditionneurs et des épaississants, des agents conservateurs, des colorants cosmétiques et alimentaires, des parfums, des arômes, des agents de régulation du pH et leurs mélanges.

L'agent gélifiant ou épaississant peut être hydrophile ou peut être de type silicone. Les gélifiants hydrophiles peuvent être des gélifiants synthétiques, semi-synthétiques, ou naturels d'origine végétale, microbienne, animale ou minérale. Les gélifiants hydrophiles peuvent être par exemple des polymères et des copolymères d'acide acrylique, des polymères réticulés acrylate d'alkyle en C₁₀-₃₀/acrylate, polyacrylamide, poloxamer, dérivés cellulosiques (esters et éthers), silices, fumée de silice, silicates,tels que silicates de magnésium-aluminium, chitine et ses dérivés, gélatine, xanthane, dextrane, gellane, carraghénanes, alginates, agar-agar, gélose, pectine, gomme d'acacia, gomme karaya, gomme adragante, gomme arabique, gomme de guar, gomme de caroube, amidon et ses dérivés, scléroglucane. La concentration en gélifiant ou épaississant est de 0,01 à 10% en poids, de préférence de 0,1 à 5% en poids, et plus préférentiellement encore de 0,5 à 3% en poids du poids total de la composition.

L'agent gélifiant de type silicone est par exemple un polysiloxanes.

L'agent tensio-actif peut être hydrophobe avec un HLB de 3 à 10 ou hydrophile avec un HLB de 11 à 18. Cet agent tensio-actif est choisi dans le groupe comprenant des alcools gras éthoxylés, des acides et des esters gras (par exemple : cétéareth-12, cétéareth-20, cétéareth-33, stéaryl cétyl alcool 20-éthoxylé, 2-polyhydroxystéarate polyglycéryl, glycéryl oléate, ester de sorbitan, ester de glycérol, PEG-mono/di-laurate, PEG-mono/di-stéarate, cétéaryl isononanoate, glycéryl stéarate, etc.), des carboxylates, des éthoxycarboxylates (par exemple : stéarate de sodium/potassium, acide alkyl-carboxylique, acide carboxylique éther alkyl-polyglycol, acide carboxylique éther polyglycol alkylphénol, alcool carboxyméthylé, éthoxycarboxylate, éthercarboxylate, etc.) et leurs mélanges. La quantité d'agent tensio-actif lorsqu'il est présent est de 0,1% à 10% en poids, de préférence de 0,5 à 5% en poids, et plus préférentiellement encore de 1 à 3% en poids du poids total de la composition.

Les cires ou huiles synthétiques ou naturelles, choisies dans le groupe comprenant extrait de Carnauba, cire d'abeille, beurre de karité, triglycérides, stéarines, esters d'acides gras (par exemple : des alcools cétéaryliques, des éthers dicapryliques de cétyl palmitate, des carbonates dicapryliques, des isononanates cétéaryliques, des dimères distéaryl-tricarbonate, etc.), des huiles de silicone, des stéarates de zinc, des polyisobutènes, des octyldodécanols, des octyldécylxylosides, des alcools gras, des acides gras (par exemple : acide laurique, acide myristique, acide stéarique, etc.), des huiles végétales (par exemple: huile de tournesol, de jojoba, de cocos nucifera, de soja, d'amande, etc.), la vaseline, la lanoline et leurs mélanges.

Les agents hydratants et émollients peuvent être choisis dans le groupe comprenant allantoïne, polyol (par exemple glycérol, polymères de glycérol, propylène glycol, sorbitol etc.), des extraits végétaux (par exemple des extraits d'aloe vera, de camomille, de concombre, de calendula, etc.), acide hyaluronique, acide pyrrolidone carboxylique, urée, chitosan, tocophérol, panthénol, butylène glycol, phospholipide, acide linoléique, acide γ-linoléique, alpha-bisabolol, et leurs mélanges.

La concentration en agents hydratants et émollients est de 0,1 à 20% en poids, de préférence de 0,5 à 10% en poids, et plus préférentiellement encore de 1 à 5% en poids du poids total de la composition.

Les agents anti-perspirants peuvent être choisis dans le groupe comprenant des sels d'aluminium, de préférence le sesqui-chlorhydrate d'aluminium, des sels d'aluminium et de zirconium, des complexes d'aluminium-zirconium octachlorohydrex glycine et leurs mélanges. La concentration en agent anti-perspirant est de 0,1 à 50% en poids, de préférence de 10 à 30% en poids, et plus préférentiellement encore de 15% en poids du poids total de la composition.

Les conditionneurs peuvent être choisis dans le groupe comprenant des polymères polycationiques désignés selon la nomenclature INCI polyquaterniums, des gommes quaternisées, des phospholipides quaternisés, et leurs mélanges. La concentration en conditionneur est de 0,1 à 20% en poids, de préférence de 0,5 à 10% en poids, et plus préférentiellement encore de 0,5 à 5% en poids du poids total de la composition.

Les agents conservateurs peuvent être choisis dans le groupe comprenant: ester para-hydroxybenzoique d'alkyle, isothiazolinone, imidazolidinyl urée, diazolidinyl urée, des bromo-nitro-propanediol, phénoxyéthanol, acide sorbique et ses sels, acide benzoique et ses sels, phénoxyéthanol, alcool benzylique, et leurs mélanges. La concentration est celle autorisée en cosmétique.

La concentration en colorants cosmétiques et alimentaires, parfums, arômes, agents de régulation du pH (par exemple, acide citrique, acide lactique, acide phosphorique, hydroxyde de sodium, potasse, amino-méthyl-propanol, triéthanolamine, etc.) et leurs mélanges, est de 0,1 à 50% en poids, de préférence de 10 à 30% en poids, et plus préférentiellement encore de 15% en poids du poids total de la composition.

De façon particulièrement intéressante, la composition est une préparation gélifiante, notamment une préparation lubrifiante.

La préparation lubrifiante est une préparation hydrophile, une préparation de silicone ou une préparation lubrifiante à base de vaseline contenant ledit agent chélatant amphotère ou bien sous forme liquide ou bien sous forme solide.

La quantité d'agent chélatant amphotère présente dans ladite composition est de 0,01 à 5%, de préférence de 0,02 à 4,4% et plus préférentiellement encore d'environ 2% en poids du poids total de la composition. Pour certaines applications, où les quantités d'allergènes sont très faibles, notamment lors de l'utilisation de latex déprotéiné, de très faibles quantités, même inférieures à 0,01% pourraient s'avérer suffisantes. Cependant, dans la généralité des cas une quantité inférieure à 0,01% ne permet pas d'obtenir un effet préventif suffisant. Au-delà de 5% on n'observe pas d'amélioration sensible.

Selon l'invention, la composition est utilisée en application topique, plus particulièrement cutanée. Elle peut également être utilisée par application sur la surface du dispositif destiné à entrer en contact avec la peau et/ou les muqueuses.

Sans vouloir être liés par aucune théorie, les présents inventeurs sont d'avis que la composition selon l'invention permet de réaliser une barrière physique et chimique contre les allergènes. Plus particulièrement, après application et séchage, une couche barrière recouvre la peau assurant ainsi une barrière physique entre la peau et l'environnement extérieur, l'agent chélatant amphotère assurant quant à lui une barrière chimique en chélatant ou en réagissant chimiquement avec les allergènes entrant en contact avec cette composition.

Par cette double action, les allergènes sont empêchés d'entrer en contact avec la peau.

Pour être efficace, la composition doit recouvrir en totalité la zone de peau et/ou de muqueuse susceptible d'entrer en contact avec le dispositif en latex, généralement les mains pour les gants en latex ou le sexe pour les préservatifs. Elle doit être appliquée sur toute la surface de contact. La quantité d'application de la composition selon l'invention est à raison d'environ 0,5 à 5 mg/cm², de préférence d'environ 1 à 3 mg/cm², et plus préférentiellement encore d'environ 1,5 mg/cm².

### EXEMPLES

Les exemples suivants décrivent certains modes de réalisation de la présente invention. Cependant, il est entendu que les exemples ne sont présentés qu'à titre illustratif seulement et ne limitent en aucun cas la portée de l'invention.

Dans les présents exemples, l'agent chélatant amphotère utilisé présente la formule suivante: [AlYBO₂]²⁻ Na⁺₂.

### EXEMPLE 1- COMPOSITION LUBRIFIANTE

On prépare une composition lubrifiante contenant :

| | |
|---|---|
| Aloe vera | 0,1 mg |
| Vaseline | 100 mg |
| Lanoline | 35 mg |
| Eau | 15 mg |
| Agent chélatant amphotère | 0,08 mg |

### EXEMPLE 2 Gel

* 49,5 mL eau
* 0,5 g gomme xanthane
* 0,2 mg d'agent chélatant amphotère

Le gel est préparé de la façon suivante : on verse l'eau dans un récipient. On agite tout en ajoutant la gomme et l'agent chélatant amphotère. On laisse reposer 10 min.

On mélange pour obtenir une texture lisse.

### EXEMPLE 3: EMULSION POUR USAGE EXTERNE

On a préparé une émulsion comprenant les produits

| | |
|---|---|
| Stéarate de glycérol / Alcool cétéarylique/ cétyl palmitate/ Glycérides de coco (ratio en poids: 60%/20%/10%/10%) | 0, 12g |
| Alcool cétéarylique éthoxylé (12 moles) | 0, 01g |
| Alcool cétéarylique éthoxylé (20 moles) | 0, 02g |
| C18- caprylate/ Caprate | 0, 09g |
| Caprylique/ caprique triglycéride | 0, 03g |
| Paraffine liquide | 0, 02g |
| Propylparaben/ benzoate paraben (50/50 p/p) | 0, 004g |
| Eau purifiée | qs p 1g |
| Glycérine | 0, 03g |
| Agent chélatant amphotère | 0, 002g |

Cette émulsion huile dans l'eau présente les caractéristiques suivantes:
- viscosité Brookfield de 18000 cP, mesurée à une température de 20(+/-1)°C et avec une vitesse de mobile à 3/20 rpm,
- pH de 6,45,
- Aucun déphasage ne s'est produit après stockage pendant 6 mois à la température ambiante.

### EXEMPLE 4 : Essai d'efficacité contre des allergènes

On prépare des explants de peau d'un diamètre moyen de 10 mm, provenant d'une abdoplastie d'une femme caucasienne de 49 ans. Ces explants sont maintenus vivants dans un milieu de culture BEM (BIO-EC explant Medium) à 37°C en atmosphère humide et enrichie de 5% de CO_{2.}

On teste la composition de l'exemple 3 contre les allergènes suivants :
- métaux lourds (Al, As, Cd, Cr, Hg, Ni, Pb, Sr, Te) - ECP multi-element standard V Ref 0C467028 Merck;
- Disulfure de tétraméthylthiuram - T2 420 ;
- 2-Mercaptobenzothiazole - M3301;
- Carbamate de Benzyle - B18200;
- Bisphénol - 13302;
- Acrylate d'éthyle - W241806;
- Araldite 506 epoxy Resin - A3183;
- Hévéine - latex 4335932 ;
- Méthanal à 30% w/w - 116 99031.

A T₀, on applique la composition de l'exemple 3 ci-dessus sur les explants de peau à un dosage de 3 mg/cm². On laisse sécher pendant 15 mn. Ensuite, 25µl d'une solution de l'un des allergènes choisis dans la liste ci-dessus sont appliqués sur les explants de peau protégés par l'émulsion de l'exemple 3 (test) et sont également appliqués sur des explants de peau non-protégés (témoin). On laisse reposer jusqu'à T₄ₕ, ce qui représente 4 heures d'exposition à l'allergène sans lavage ni friction abrasive.

Parallèlement on prépare de la même façon des explants protégés par l'émulsion mais qui ne sont pas mis en contact avec une substance à tester (contrôle).

L'efficacité de protection contre des allergènes est mesurée par l'analyse d'immuno-coloration de CD1a. D'abord, les sections paraffinées de cellules Langerhans sont immuno-colorées avec des anticorps monoclonaux anti-CD1a (ref. IM1590, clone O10, Beckman Coulter) pendant 1 heure à température ambiante. Cette immuno-coloration est renforcée par un système de streptavidine/biotine (Vector, PK-7200) et révélée en employant VIP (Vector, SK-4600). Les nucléus sont contre-colorés par hémalum de Masson. Les cellules Langerhans sont comptées sur chaque section le long d'épiderme. La longueur de chaque section est mesurée par le logiciel Olympus Cell et le nombre moyen de cellules Langerhans par centimètre d'épiderme est calculé.

L'efficacité de protection contre l'allergène est mesurée par le nombre de cellules Langerhans n'ayant pas migré.

Des échantillons sont prélevés pour tous les explants à T₀ et à T₄ₕ et sont photographiés.

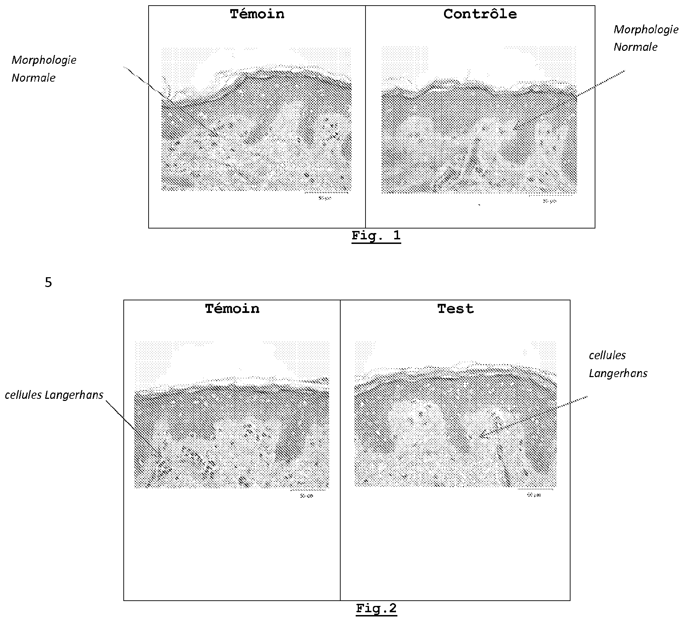

Les résultats sont présentés sur les figures 1 et 2. La figure 1 représente les explants à Tₒ et la figure 2 à T₄ₕ pour l'Hévéine.

A T₀, le stratum corneum est fin, compact et modérément rempli de kératine en surface et remarquablement en bas. L'épiderme présente de 4 à 5 couches cellulaires avec une bonne morphologie et une spongiose légère sur le stratum germinativum. Le relief de jonction entre le derme et l'épiderme est marqué. Le derme papillaire présente des fibres assez épaisses construisant un réseau dense et il est bien cellularisé sans zone de lyse histologique apparente.

L'épiderme des explants non-protégés présente 4-5 couches cellulaires avec des changements morphologiques remarquables. Ces changements sont caractérisés par une dénaturation cytoplasmique marquée (altération protéique), une pycnose marquée et une spongiose marquée sur le stratum germinativum.

En revanche, les explants protégés et "contrôle" ne montrent aucun changement histologique significatif.

Des résultats analogues ont été obtenus avec chacun des autres allergènes testés.

L'absence ou le bas niveau de changement cellulaire ainsi que la concentration faible d'interleukines ont démontré que la composition de l'exemple 3 protège la peau efficacement contre l'hévéine.

### EXEMPLE 5 : composition pour préservatif

On prépare de la façon suivante 100 mL de gel lubrifiant comprenant :
- 99 mL d'eau purifiée
- 0,5 g de scléroglucane
- 0,4 mg d'agent chélatant amphotère

Ce gel est appliqué par enduction sur les faces internes et externes d'un préservatif en caoutchouc naturel en une quantité de 2,0 +/- 0,5 mg/cm².

## Revendications

1. Agent chélatant amphotère qui comprend un complexe à base d'aluminium et d'acide éthylène diamine tétraacétique ou son sel trisodique présentant la formule générale [Al(Y)Bₙ]^{c'}D_{c} avec B représentant OH⁻, BO₂⁻, H⁺, Y représentant un tétracarboxylate pouvant être protoné quatre fois pour former l'acide éthylène diamine tétraacétique, n représentant un nombre entier égal à 0, 1, 2 ou 3, D étant un contre-ion, de préférence Na⁺, c étant un nombre entier égal à 0, 1, 2 ou 3 et c' étant un nombre relatif ayant la même valeur absolue que c, pour une utilisation pour prévenir les allergies de contact qui sont provoquées par le contact répété avec la peau et/ou les muqueuses de substances allergènes, en particulier les allergies au caoutchouc, ledit agent chélatant amphotère étant stabilisé par un acide aminé choisi dans le groupe consistant en la glycine, l'histidine, l'arginine, la phénylalanine, l'isoleucine, la leucine, la méthionine, la proline, la valine, le tryptophane, la sérine, la glutamine, la cystine et leurs mélanges.

2. Agent chélatant amphotère pour une utilisation selon la revendication 1, **caractérisé par le fait que** l'allergie de contact est une allergie à une substance allergène, une dermite de contact, une urticaire de contact, eczéma, rhinites, conjonctivites, asthme, choc anaphylactique.

3. Agent chélatant amphotère pour une utilisation selon la revendication 1 ou 2, dans laquelle ledit agent chélatant est en contact avec la peau et/ou les muqueuses.

4. Agent chélatant amphotère pour une utilisation selon l'une quelconque des revendications 1 à 3, dans laquelle le composé amphotère est incorporé dans un dispositif et/ou dans une composition.

5. Dispositif comportant une partie en matériau contenant des agents allergènes qui est destinée à entrer en contact avec la peau ou les muqueuses, ladite partie étant recouverte par une couche contenant au moins un agent chélatant amphotère qui comprend un complexe à base d'aluminium et d'acide éthylène diamine tétraacétique ou son sel trisodique présentant la formule générale [Al(Y)Bₙ]^{c'}D_{c} avec B représentant OH⁻, BO₂⁻, H⁺, Y représentant un tétracarboxylate pouvant être protoné quatre fois pour former l'acide éthylène diamine tétraacétique, n représentant un nombre entier égal à 0, 1, 2 ou 3, D étant un contre-ion, de préférence Na⁺, c étant un nombre entier égal à 0, 1, 2 ou 3 et c' étant un nombre relatif ayant la même valeur absolue que c.

6. Dispositif selon la revendication 5, choisi parmi les préservatifs, les gants, les doigtiers, les pansements et compresses, les dispositifs médicaux tels que sondes, tubulures, dispositifs de drainage, de perfusion, dispositifs médicaux utiles en urologie, dispositifs médicaux de l'abord respiratoire.

7. Dispositif selon la revendication 5 ou 6, dans lequel l'agent chélatant amphotère est présent dans un film déposé au moins sur la surface destinée à entrer en contact avec la peau et/ou les muqueuses.

8. Composition comprenant au moins un agent chélatant amphotère qui comprend un complexe à base d'aluminium et d'acide éthylène diamine tétraacétique ou son sel trisodique présentant la formule générale [Al(Y)Bₙ]^{c'}D_{c} avec B représentant OH⁻, BO₂⁻, H⁺, Y représentant un tétracarboxylate pouvant être protoné quatre fois pour former l'acide éthylène diamine tétraacétique, n représentant un nombre entier égal à 0, 1, 2 ou 3, D étant un contre-ion, de préférence Na⁺, c étant un nombre entier égal à 0, 1, 2 ou 3 et c' étant un nombre relatif ayant la même valeur absolue que c, et au moins un excipient, pour une utilisation pour prévenir les allergies de contact qui sont provoquées par le contact répété avec la peau et/ou les muqueuses de substances allergènes, en particulier les allergies au caoutchouc, **caractérisée en ce que** ledit agent chélatant amphotère est stabilisé par un acide aminé choisi dans le groupe consistant en la glycine, l'histidine, l'arginine, la phénylalanine, l'isoleucine, la leucine, la méthionine, la proline, la valine, le tryptophane, la sérine, la glutamine, la cystine et leurs mélanges.

9. Composition pour une utilisation selon la revendication 8, **caractérisée par le fait que** ledit excipient est choisi parmi un agent gélifiant, des tensio-actifs, des cires ou huiles synthétiques ou naturelles, des agents hydratants et émollients, des agents anti-perspirants, des additifs améliorant la texture tels que des conditionneurs , des agents conservateurs, des colorants cosmétiques et alimentaires, des parfums, des arômes, des agents de régulation du pH et leurs mélanges.

10. Composition pour une utilisation selon la revendication 9, **caractérisée par le fait que** l'agent gélifiant est choisi parmi les gélifiants hydrophiles tels que des polymères et des copolymères d'acide acrylique, des polymères réticulés acrylate d'alkyle en C₁₀-₃₀/acrylate, polyacrylamide, poloxamer, dérivés cellulosiques(esters et éthers), silices, silicates tels que silicates de magnésium-aluminium, chitine et ses dérivés, gélatine, xanthane, dextrane, gellane, carraghénanes, alginates, agar-agar, gélose, pectine, gomme d'acacia, gomme karaya, gomme adragante, gomme arabique, gomme de guar, gomme de caroube, amidon et ses dérivés, scléroglucane ; les agents gélifiants de type silicone, tels qu'un polysiloxane.

11. Composition pour une utilisation selon l'une quelconque des revendications 8 à 10, **caractérisée par le fait que** la composition est une préparation lubrifiante choisie parmi une préparation lubrifiante hydrophile, une préparation lubrifiante de silicone ou une préparation lubrifiante à base de vaseline.

12. Dispositif selon l'une quelconque des revendications 5 à 7, **caractérisé par le fait que** l'agent chélatant amphotère est stabilisé par un acide aminé choisi dans le groupe comprenant la glycine, l'histidine, l'arginine, la lysine, la phénylalanine, l'alanine, l'isoleucine, la leucine, la méthionine, la proline, la valine, le tryptophane, la sérine, la glutamine, la cystine et leurs mélanges.

## Patentansprüche

1. Amphoterer Chelatbildner, umfassend einen Komplex aus Aluminium und Ethylendiamintetraessigsäure oder dessen Trinatrium-Salz, der durch die allgemeine Formel [Al(Y)Bₙ]^{c'}D_{c} dargestellt ist, wobei B für OH⁻, BO₂⁻, H⁺ steht, Y für ein Tetracarboxylat steht, das viermal protoniert sein kann, um Ethylendiamintetraessigsäure zu bilden, n für eine ganze Zahl gleich 0, 1, 2 oder 3 steht, D ein Gegenion, vorzugsweise Na⁺, ist, c eine ganze Zahl gleich 0, 1, 2, oder 3 ist und c' eine relative Zahl mit dem gleichen Betrag wie c ist,
zur Verwendung zur Vermeidung von Kontaktallergien, die durch wiederholten Kontakt der Haut und/oder der Schleimhäute mit allergenen Stoffen ausgelöst werden, insbesondere Kautschukallergien,
wobei der amphotere Chelatbildner durch eine Aminosäure stabilisiert wird, die ausgewählt ist aus der Gruppe bestehend aus Glycin, Histidin, Arginin, Phenylalanin, Isoleucin, Leucin, Methionin, Prolin, Valin, Tryptophan, Serin, Glutamin, Cystin und Mischungen davon.

2. Amphoterer Chelatbildner zur Verwendung gemäß Anspruch 1, **dadurch gekennzeichnet, dass** die Kontaktallergie eine Allergie gegen einen allergenen Stoff, Kontaktdermatitis, Kontakt-Nesselsucht, Ekzem, Rhinitis, Konjunktivitis, Asthma, Anaphylaxie ist.

3. Amphoterer Chelatbildner zur Verwendung gemäß Anspruch 1 oder 2, wobei der Chelatbildner mit der Haut und/oder den Schleimhäuten in Kontakt steht.

4. Amphoterer Chelatbildner zur Verwendung gemäß einem der Ansprüche 1 bis 3, wobei die amphotere Verbindung in einer Vorrichtung und/oder einer Zusammensetzung enthalten ist.

5. Vorrichtung, umfassend einen Materialabschnitt, der Allergene enthält, der dazu bestimmt ist mit der Haut oder den Schleimhäuten in Kontakt zu kommen, wobei der Abschnitt mit einer Schicht überzogen ist, die mindestens einen amphoteren Chelatbildner, umfassend einen Komplex aus Aluminium und Ethylendiamintetraessigsäure oder dessen Trinatrium-Salz, enthält, der durch die allgemeine Formel [Al(Y)Bₙ]^{c'}D_{c} dargestellt ist, wobei B für OH⁻, BO₂⁻, H⁺ steht, Y für ein Tetracarboxylat steht, das viermal protoniert sein kann, um Ethylendiamintetraessigsäure zu bilden, n für eine ganze Zahl gleich 0, 1, 2 oder 3 steht, D ein Gegenion, vorzugsweise Na⁺, ist, c eine ganze Zahl gleich 0, 1, 2, oder 3 ist und c' eine relative Zahl mit dem gleichen Betrag wie c ist.

6. Vorrichtung gemäß Anspruch 5, die ausgewählt ist aus Präservativen, Handschuhen, Fingerlingen, Verbänden und Kompressen, medizinischen Vorrichtungen wie Sonden, Schläuche, Drainagevorrichtungen, Infusionsvorrichtungen, medizinischen Vorrichtungen zur Verwendung in der Urologie, medizinischen Beatmungsvorrichtungen.

7. Vorrichtung gemäß Anspruch 5 oder 6, in der der amphotere Chelatbildner in einem Film vorliegt, der mindestens auf der Oberfläche aufgebracht ist, die dazu bestimmt ist mit der Haut und/oder den Schleimhäuten in Kontakt zu kommen.

8. Zusammensetzung, umfassend mindestens einen amphoteren Chelatbildner, umfassend einen Komplex aus Aluminium und Ethylendiamintetraessigsäure oder dessen Trinatrium-Salz, der durch die allgemeine Formel [Al(Y)Bₙ]^{c'}D_{c} dargestellt ist, wobei B für OH⁻, BO₂⁻, H⁺ steht, Y für ein Tetracarboxylat steht, das viermal protoniert sein kann, um Ethylendiamintetraessigsäure zu bilden, n für eine ganze Zahl gleich 0, 1, 2 oder 3 steht, D ein Gegenion, vorzugsweise Na⁺, ist, c eine ganze Zahl gleich 0, 1, 2, oder 3 ist und c' eine relative Zahl mit dem gleichen Betrag wie c ist, und mindestens einen Hilfsstoff,
zur Verwendung zur Vermeidung von Kontaktallergien, die durch wiederholten Kontakt der Haut und/oder der Schleimhäute mit allergenen Stoffen ausgelöst werden, insbesondere Kautschukallergien,
**dadurch gekennzeichnet, dass** der amphotere Chelatbildner durch eine Aminosäure stabilisiert wird, die ausgewählt ist aus der Gruppe bestehend aus Glycin, Histidin, Arginin, Phenylalanin, Isoleucin, Leucin, Methionin, Prolin, Valin, Tryptophan, Serin, Glutamin, Cystin und Mischungen davon.

9. Zusammensetzung zur Verwendung gemäß Anspruch 8, **dadurch gekennzeichnet, dass** der Hilfsstoff ausgewählt ist aus einem Gelierungsmittel, Tensiden, Wachsen oder synthetischen oder natürlichen Ölen, hydratisierenden und hautaufweichenden Mitteln, Antitranspirantien, Zusätzen zur Verbesserung der Beschaffenheit, wie Konditionierungsmittel, Konservierungsmittel, kosmetische Farbstoffe und Lebensmittelfarbstoffe, Duftstoffe, Aromen, pH-Regulierungsmittel und Mischungen davon.

10. Zusammensetzung zur Verwendung gemäß Anspruch 9, **dadurch gekennzeichnet, dass** das Gelierungsmittel ausgewählt ist aus hydrophilen Gelierungsmitteln wie Polymere und Copolymere der Acrylsäure, vernetzte Alkylacrylatpolymere von C₁₀₋₃₀-Alkyl/Acrylat, Polyacrylamid, Poloxamer, Cellulosederivate (Ester und Ether), Kieselsäuren, Silikate wie Magnesium-Aluminium-Silikate, Chitin und Derivate davon, Gelatine, Xanthan, Dextran, Gellan, Carrageene, Alginate, Agar-Agar, Agar, Pektin, Akaziengummi, Karayagummi, Traganthgummi, Gummiarabikum, Guargummi, Johannisbrotkernmehl, Stärke und Derivate davon, Scleroglucan; Gelierungsmittel des Silikontyps, wie Polysiloxane.

11. Zusammensetzung zur Verwendung gemäß einem der Ansprüche 8 bis 10, **dadurch gekennzeichnet, dass** die Zusammensetzung eine Schmiermittelzubereitung ist, die ausgewählt ist aus einer hydrophilen Schmiermittelzubereitung, einer Silikonschmiermittelzubereitung oder einer Schmiermittelzubereitung auf Vaselinbasis.

12. Vorrichtung gemäß einem der Ansprüche 5 bis 7, **dadurch gekennzeichnet, dass** der amphotere Chelatbildner durch eine Aminosäure stabilisiert wird, die ausgewählt ist aus der Gruppe bestehend aus Glycin, Histidin, Arginin, Lysin, Phenylalanin, Alanin, Isoleucin, Leucin, Methionin, Prolin, Valin, Tryptophan, Serin, Glutamin, Cystin und Mischungen davon.

## Claims

1. Amphoteric chelating agent which comprises a complex based on aluminum and on ethylenediaminetetraacetic acid or the trisodium salt thereof, having the general formula [Al(Y)Bₙ]^{c'}D_{c} with B being OH⁻, BO₂⁻, H⁺, Y being a tetracarboxylate which can be protonated four times so as to form ethylenediaminetetraacetic acid, n being an integer equal to 0, 1, 2 or 3, D being a counterion, preferably Na⁺, c being an integer equal to 0, 1, 2 or 3 and c' being a relative number having the same absolute value as c, for use to prevent contact allergies, which are caused by repeated contact of allergenic substances with the skin and/or the mucous membranes, in particular allergies to rubber, said amphoteric chelating agent being stabilized with an amino acid chosen from the group consisting in glycine, histidine, arginine, phenylalanine, isoleucine, leucine, methionine, proline, valine, tryptophan, serine, glutamine, cystine, and mixtures thereof.

2. Amphoteric chelating agent for the use as claimed in claim 1, **characterized in that** the contact allergy is an allergy to an allergenic substance, contact dermatitis, contact urticaria, eczema, rhinitis, conjunctivitis, asthma, or anaphylactic shock.

3. Amphoteric chelating agent for the use as claimed in claim 1 or 2, wherein said chelating agent is in contact with the skin and/or the mucous membranes.

4. Amphoteric chelating agent for the use as claimed in any one of claims 1 to 3, wherein the amphoteric compound is incorporated into a device and/or into a composition.

5. A device comprising a part made of material containing allergenic agents, which is intended to come into contact with the skin or the mucous membranes, said part being covered with a layer containing at least one amphoteric chelating agent which comprises a complex based on aluminum and on ethylenediaminetetraacetic acid or the trisodium salt thereof, having the general formula [Al(Y)Bₙ]^{c'}D_{c} with B being OH⁻, BO₂⁻, H⁺, Y being a tetracarboxylate which can be protonated four times so as to form ethylenediaminetetraacetic acid, n being an integer equal to 0, 1, 2 or 3, D being a counterion, preferably Na⁺, c being an integer equal to 0, 1, 2 or 3 and c' being a relative number having the same absolute value as c.

6. The device as claimed in claim 5, chosen from condoms, gloves, finger stalls, bandage and compresses, medical devices such as catheters, tubing, draining devices, perfusion devices, medical devices of use in urology, and medical devices for respiratory access.

7. The device as claimed in claim 5 or 6, wherein the amphoteric chelating agent is present in a film deposited at least on the surface intended to come into contact with the skin and/or the mucous membranes.

8. Composition comprising at least one amphoteric chelating agent which comprises a complex based on aluminum and on ethylenediaminetetraacetic acid or the trisodium salt thereof, having the general formula [Al(Y)Bₙ]^{c'}D_{c} with B being OH⁻, BO₂⁻, H⁺, Y being a tetracarboxylate which can be protonated four times so as to form ethylenediaminetetraacetic acid, n being an integer equal to 0, 1, 2 or 3, D being a counterion, preferably Na⁺, c being an integer equal to 0, 1, 2 or 3 and c' being a relative number having the same absolute value as c, and at least one excipient, for use to prevent contact allergies, which are caused by repeated contact of allergenic substances with the skin and/or the mucous membranes, in particular allergies to rubber, **characterized in that** said amphoteric chelating agent is stabilized with an amino acid chosen from the group consisting in glycine, histidine, arginine, phenylalanine, isoleucine, leucine, methionine, proline, valine, tryptophan, serine, glutamine, cystine, and mixtures thereof.

9. Composition for the use as claimed in claim 8, **characterized in that** said excipient is chosen from a gelling agent, surfactants, synthetic or natural waxes or oils, moisturizing agents and emollients, antiperspirants, texture-improving additives such as conditioners, preservatives, cosmetic and food dyes, fragrances, flavorings, pH-regulating agents, and mixtures thereof.

10. Composition for the use as claimed in claim 9, **characterized in that** the gelling agent is chosen from hydrophilic gelling agents, such as acrylic acid polymers and copolymers, C₁₀-C₃₀ alkyl acrylate/acrylate crosslinked polymers, polyacrylamide, poloxamer, cellulose-based derivatives (esters and ethers), silicas, silicates, such as magnesium aluminum silicates, chitin and derivatives thereof, gelatin, xanthan, dextran, gellan, carraghenans, alginates, agar-agar, agar, pectin, acacia gum, karaya gum, tragacanth, gum arabic, guar gum, locust bean gum, starch and derivatives thereof, or scleroglucan; and gelling agents of silicone type, such as a polysiloxane.

11. Composition for the use as claimed in any one of claims 8 to 10, **characterized in that** the composition is a lubricating preparation chosen from a hydrophilic lubricating preparation, a silicone lubricating preparation or a vaseline-based lubricating preparation.

12. The device as claimed in any one of claims 5 to 7, **characterized in that** the amphoteric chelating agent is stabilized with an amino acid chosen from the group comprising glycine, histidine, arginine, lysine, phenylalanine, alanine, isoleucine, leucine, methionine, proline, valine, tryptophan, serine, glutamine, cystine, and mixtures thereof.
